# EUROPEAN PATENT APPLICATION

(11) **EP 4 631 493 A1**
(43) Date of publication of application: **15.10.2025**
(21) Application number: 23900750.3
(22) Date of filing: 08.12.2023
(51) Int. Cl.: A61K 8/891, A61Q 1/02, A61Q 1/04, A61Q 1/10, A61Q 17/04, A61Q 19/00

(54) **COSMETIC**

(30) Priority: 08.12.2022 JP 2022196467
(71) Applicant: Momentive Performance Materials Japan LLC, Tokyo 107-6119 (JP)
(72) Inventor: HORIE Yutaka, Tokyo 107-6119 (JP); LI Qinghua, Tokyo 107-6119 (JP)
(74) Representative: Gille Hrabal Partnerschaftsgesellschaft mbB Patentanwälte
(86) International application number: PCT/JP2023/044037
(87) International publication number: WO 2024/122638

(57) **Abstract**

To provide a cosmetic preparation attaining excellent usability by spreading well, having no sticky feeling, and providing a soft coating, the preparation containing the following components (A) and (B):
(A) one or two or more selected from 1,1,1,3,5,5,5-heptamethyl-3-alkyl (C10-14) trisiloxanes; and
(B) a silicone resin having an average unit formula of RₐSiO_{(4-a)/2} and a thermal softening point of
50°C or more, wherein R represents the same or different substituted or unsubstituted monovalent hydrocarbon groups, and a is a number from 1.2 to 2.

## Description

### Field of the Invention

The present invention relates to a cosmetic preparation that can be used as skin cosmetic preparations or makeup cosmetic preparations.

### Background of the Invention

In recent years, the demands on cosmetic preparations have continued to diversify, and in response thereto, their product forms have also become wide-ranging, including not only elements in terms of an item lineup such as foundations, cosmetic primers, sunscreens, lipsticks, nail polishes, face powders, and others, but also those in terms of forms such as liquid form, powder form, solid form, emulsion form, and others as subcategories of the above.

In particular, in addition to water resistance or sweat resistance (sebum resistance) more than before, good ease of use during application or various physical/chemical properties such as rub resistance and others have been required of recent cosmetic preparations.

In order to meet the above requirements, as a technology using a silicone resin having a coating-forming ability, a skin cosmetic preparation of JP-A S61-158910, an additive for makeup cosmetic preparations of JP-A H2-42008, a cosmetic preparation of JP-A H4-312511, and a cosmetic preparation of WO-A 2009/136486 have been proposed.

Further, JP-A H9-12431 has proposed an invention of a cosmetic preparation that contains a fluorine group-containing silicone resin, the preparation being excellent in water resistance or sweat resistance and providing a soft coating.

### Summary of the Invention

The problem of the present invention is to provide a cosmetic preparation that attains excellent usability by spreading well, having no sticky feeling, and providing a soft coating.

The present invention provides a cosmetic preparation containing the following components (A) and (B) :
(A) one or two or more selected from 1,1,1,3,5,5,5-heptamethyl-3-alkyl (C10-14) trisiloxanes; and
(B) a silicone resin having an average unit formula of RₐSiO_{(4-a)/2} and a thermal softening point of 50°C or more, wherein R represents a substituted or unsubstituted monovalent hydrocarbon group, and a is a number from 1.2 to 2.

The cosmetic preparation of the present invention spreads well and has softness to the touch when applied to the skin, and is useful for preventing makeup from coming off due to sweat or the like. Further, the cosmetic preparation of the present invention can have a good usage characteristic that it is not easily washed off even by rinsing with water or the like or good stability even over time.

### Embodiments of the Invention

The component (A) is one or two or more selected from 1,1,1,3,5,5,5-heptamethyl-3-alkyl (C10-14) trisiloxanes. This may be in oil form at ordinary temperatures.

Non-limiting examples of the component (A) can include (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane. While these components (A-1), (A-2), and (A-3) can be each used alone as the component (A), the component (A-2) is preferably used if the component (A) is used as a single component.

A mixture of the components (A-1) and (A-2), a mixture of the components (A-1) and (A-3), a mixture of the components (A-2) and (A-3), or a mixture of the components (A-1), (A-2), and (A-3) can be used as the component (A).

If the component (A) is used as the mixture of the components (A-1) and (A-2) or the mixture of the components (A-2) and (A-3), a content of the component (A-2) contained in 100 mass% of the mixture is preferably 50 mass% or more, more preferably 60 mass% or more, and further preferably 70 mass% or more.

If the component (A) is used as the mixture of the components (A-1) and (A-3), an amount of the component (A-1) in a total amount of 100 mass% of the components (A-1) and (A-3) is preferably 20 to 80 mass%, more preferably 30 to 70 mass%, and further preferably 40 to 60 mass%. In this case, an amount of the component (A-3) is preferably 80 to 20 mass%, more preferably 70 to 30 mass%, and further preferably 60 to 40 mass%.

If the component (A) is used as the mixture of the components (A-1), (A-2), and (A-3), an amount of the component (A-1) in a total of 100 mass% of the components (A-1), (A-2), and (A-3) is preferably 5 to 80 mass%, more preferably 10 to 70 mass%, and further preferably 20 to 60 mass%. In this case, an amount of the component (A-2) is preferably 10 to 70 mass%, more preferably 20 to 60 mass%, and further preferably 20 to 50 mass%. Further, in this case, an amount of the component (A-3) is preferably 10 to 70 mass%, more preferably 20 to 60 mass%, and further preferably 20 to 50 mass%.

The silicone resin of the component (B) has an average unit formula of RₐSiO_{(4-a)/2} and a thermal softening point of 50°C or more, wherein R represents the same or different substituted or unsubstituted monovalent hydrocarbon groups, and a is a number from 1.2 to 2.

Examples of the substituted or unsubstituted monovalent hydrocarbon group of R in the above constituent unit can include, for example, a group having 1 to 12, particularly 1 to 8 carbons in the portion excluding a substituent. Non-limiting examples of the substituted or unsubstituted monovalent hydrocarbon group of R include alkyl groups such as a methyl group, an ethyl group, a propyl group, a butyl group, and others, alkenyl groups such as a vinyl group, an allyl group, and others, aryl groups such as a phenyl group, a tolyl group, and others, cycloalkyl groups such as a cyclohexyl group, a cyclooctyl group, and others, or groups formed by the substitution of a halogen atom, a cyano group, an amino group, or the like for a hydrogen atom bonded to a carbon atom of these groups, a cyanomethyl group, a γ-aminopropyl group, an N-(β-aminoethyl)-γ-aminopropyl group, and others. A methyl group, an ethyl group, or a phenyl group is preferable for ease of synthesis and water repellency.

If a is less than 1.2 in the above average unit formula, a coating formed after the cosmetic preparation is applied to the skin tends to be hard, and may have effects on feelings to the touch during use of the cosmetic preparation, such as tightness, stiffness, and others. On the other hand, when a is more than 2 in the above average unit formula, the above coating tends to be viscous, and may have effects on feelings to the touch, such as greasiness, stickiness, and others. Further, in both cases, it is difficult to obtain sufficient coating durability.

Specifically, the silicone resin of the component (B) is preferably a silicone resin composed of a combination of R₃SiO_{1/2} unit, R₂SiO_{2/2} unit, RSiO_{3/2} unit, and SiO_{4/2} unit. The silicone resin resulting from this combination of the four units may have the average unit formula RₐSiO_{(4-a)/2} in which a is from 1.2 to 2. From the viewpoint of obtaining sufficient coating durability, a content of R₂SiO_{2/2} unit as a constituent in the silicone resin of the component (B) is preferably 30 mol% or less.

A mixture of multiple silicone resins that satisfies the above average unit formula may be used as the silicone resin of the component (B). Using such a mixture is preferable as it makes it easy to adjust the coating strength, durability, and suitable usability of the cosmetic preparation to be obtained.

When the mixture of multiple silicone resins that satisfies the above average unit formula is used as the silicone resin of the component (B), a silicone resin composed mainly of R₃SiO_{1/2} unit and RSiO_{3/2} unit and a silicone resin composed mainly of R₃SiO_{1/2} unit and SiO_{4/2} unit can be used together at a ratio of 1/4 to 4/1.

If the silicone resin of the component (B) has a thermal softening point of less than 50°C, the coating formed after the cosmetic preparation is applied to the skin has a sticky feeling to make the preparation inferior in usability. The thermal softening point can be measured on the basis of the method for measuring thermal softening point of JIS K 2207 (ring-and-ball method). The silicone resin of the component (B) has a thermal softening point of preferably 60°C or more and more preferably 70°C or more.

The cosmetic preparation of the present invention contains the components (A) and (B) in an amount of preferably 1 to 50 mass%, more preferably 3 to 30 mass%, and further preferably 3 to 20 mass% in total.

In a total content of 100 mass% of the components (A) and (B) in the cosmetic preparation of the present invention, a content of the component (A) is preferably 50 to 95 mass% and more preferably 60 to 90 mass%, and a content of the component (B) is preferably 5 to 50 mass% and more preferably 10 to 40 mass%.

In addition to the components (A) and (B), components commonly used in cosmetic preparations may be further added to the cosmetic preparation of the present invention depending on the type of the cosmetic preparation. For example, each of the following components can be appropriately selected and contained in the cosmetic preparation of the present invention.

The cosmetic preparation of the present invention can contain an oily component, and examples include, but are not limited to, solid paraffin, ceresin, beeswax, carnauba wax, wood wax, candelilla wax, stearic acid, cetanol, cholesterol, microcrystalline wax, petroleum jelly, lanolin, partially hydrogenated oils, squalene, liquid paraffin, castor oils, diglycerides, triglycerides, higher fatty acids, higher alcohols, perfluoropolyethers, perfluorodecalin, perfluorooctane, ester oils, octyldodecyl myristate, octyldodecyl oleate, neopentyl glycol dioctanoate, or combinations of these, and others.

The cosmetic preparation of the present invention can contain an aqueous component, and examples include, but are not limited to, glycerin, sorbitol, propylene glycol, 1,3-butylene glycol, polyethylene glycol, carboxyvinyl polymer, cellulose derivatives, sodium alginate, ethanol, or combinations of these, and others.

The cosmetic preparation of the present invention can contain a surfactant, and examples include, but are not limited to, one or a combination of two or more selected from the following groups,
the following anionic emulsifiers: sodium alkyl benzene sulfonates such as sodium hexyl benzene sulfonate, sodium octyl benzene sulfonate, sodium decyl benzene sulfonate, sodium dodecyl benzene sulfonate, sodium cetyl benzene sulfonate, and sodium myristyl benzene sulfonate; sodium alkyl naphthyl sulfonates such as sodium butyl naphthyl sulfonate; polyoxyethylene monoalkyl ether sulfate sodium salts such as sodium polyoxyethylene octyl ether sulfate, sodium polyoxyethylene decyl ether sulfate, and sodium polyoxyethylene icosyl ether sulfate; polyoxyethylene mono(alkylphenyl)ether sulfate sodium salts such as sodium polyoxyethylene decylphenyl ether sulfate; or combinations of these, and others,
the following cationic emulsifiers: quaternary ammonium salts such as octyl trimethyl ammonium chloride, dodecyl trimethyl ammonium chloride, hexadecyl trimethyl ammonium chloride, beef tallow trimethyl ammonium chloride, coconut oil trimethyl ammonium chloride, octyl dimethyl benzyl ammonium chloride, decyl dimethyl benzyl ammonium chloride, and dioctadecyl dimethyl ammonium chloride; or combinations of these, and others, and
the following nonionic emulsifiers: glycerin fatty acid esters such as glyceryl monolaurate, glyceryl monomyristate, glyceryl monostearate, and glyceryl monooleate; polyglycerin fatty acid esters, sorbitan fatty acid esters, polyoxyethylene glycerin fatty acid esters, polyoxyethylene sorbitan fatty acid esters, and polyoxyethylene fatty acid esters having a lauric acid residue, a myristic acid residue, a stearic acid residue, or an oleic acid residue; polyoxyethylene alkyl ethers such as polyoxyethylene lauryl ether, polyoxyethylene cetyl ether, polyoxyethylene stearyl ether, and polyoxyethylene oleyl ether; or combinations of these, and others.

The cosmetic preparation of the present invention can further contain a powder, and examples include, but are not limited to the following: inorganic powders such as talc, kaolin, sericite, white mica, synthetic mica, golden mica, red mica, black mica, lithia mica, vermiculite, magnesium carbonate, calcium carbonate, diatomaceous earth, magnesium silicate, calcium silicate, aluminum silicate, barium silicate, barium sulfate, strontium silicate, metal salts of tungstic acid, silica, hydroxyapatite, zeolite, boron nitride, ceramic powder, and others; organic powders such as nylon powder, polyethylene powder, benzoguanamine powder, ethylene tetrafluoride powder, distyrene benzene pinhole polymer powder, microcrystalline cellulose, and others; powders of silicone rubber and silicone resin; inorganic white pigments such as titanium oxide, zinc oxide, and others; inorganic red pigments such as an iron oxide (iron oxide red), iron titanate, and others; inorganic brown pigments such as γ-iron oxide and others; inorganic yellow pigments such as yellow iron oxide, yellow ocher, and others; inorganic black pigments such as black iron oxide, carbon black, and others; inorganic purple pigments such as mango violet, cobalt violet, and others; inorganic green pigments such as chromium oxide, chromium hydroxide, cobalt titanate, and others; inorganic blue pigments such as ultramarine, Prussian blue, and others; pearl pigments such as titanium oxide coated mica, titanium oxide coated bismuth oxychloride, bismuth oxychloride, titanium oxide coated talc, fish scale flakes, colored titanium oxide coated mica, and others; metal powder pigments such as aluminum powder, copper powder, and others; organic pigments such as Red No.201, Red No.202, Red No.204, Red No.205, Red No.220, Red No.226, Red No.228, Red No.405, Orange No.203, Orange No.204, Yellow No.205, Yellow No.401, and Blue No.404, and others; organic pigments such as zirconium, barium, or aluminum lakes of Red No.3, Red No.104, Red No.106, Red No.227, Red No.203, Red No.401, Red No.505, Orange No.205, Yellow No.4, Yellow No.5, Yellow No.202, Yellow No.203, Green No.3, and Blue No.1, and others; natural pigments such as chlorophyll, β-carotene, and others; or combinations of these, and others.

The cosmetic preparation of the present invention can contain other components in the range that the problem or the effects of the present invention are not impaired, and examples include, but are not limited to, coloring agents such as organic pigments, organic dyes, and others, preservatives, antioxidants, colorants, thickeners, pH adjusters, fragrances, UV absorbers, moisturizers, blood circulation promoters, cooling agents, antiperspirants, bactericides, skin activators, and others.

Further, the cosmetic preparation of the present invention can also contain a desired amount of water depending on the type or the formulation of the cosmetic preparation.

The cosmetic preparation of the present invention can further contain one or more selected from oil components such as various types of hydrocarbons, higher fatty acids, oils and fats, esters, higher alcohols, waxes, silicone oils, and others, such as, for example, squalane, liquid paraffin, isoparaffin, petroleum jelly, microcrystalline wax, ozokerite, ceresin, myristic acid, palmitic acid, stearic acid, oleic acid, isostearic acid, cetyl alcohol, stearyl alcohol, hexadecyl alcohol, oleyl alcohol, cetyl-2-ethylhexanoate, 2-ethylhexyl palmitate, 2-octyldodecyl myristate, 2-octyldodecyl gum ester, neopentyl glycol-2-ethylhexanate, isooctyl acid triglyceride, 2-octyldodecyl oleate, isopropyl myristate, isopropyl palmitate, isostearic acid triglyceride, coconut oil fatty acid triglycerides, olive oil, avocado oil, beeswax, myristyl myristate, mink oil, lanolin, and others, UV absorbers, antioxidants, preservatives, surfactants, moisturizers, fragrances, water, alcohols, thickeners, and others depending on the type of the cosmetic preparation, but examples are not limited to the above.

The cosmetic preparation of the present invention is preferably in any of liquid formulations, powder formulations, solid formulations, and emulsion formulations.

The cosmetic preparation of the present invention is preferably a skin cosmetic preparation or a makeup cosmetic preparation selected from cosmetic primers, foundations, sunscreens, lipsticks, eyeliners, mascaras, and skin creams.

The cosmetic preparation of the present invention encompasses each of the following inventions.
(1) A cosmetic preparation containing the following components (A) and (B):
   (A) one or two or more selected from 1,1,1,3,5,5,5-heptamethyl-3-alkyl (C10-14) trisiloxanes; and
   (B) a silicone resin having an average unit formula of RₐSiO_{(4-a)/2} and a thermal softening point of 50°C or more, wherein R represents a substituted or unsubstituted monovalent hydrocarbon group which may be the same or different, and a is a number from 1.2 to 2.
(2) The cosmetic preparation according to (1), wherein the component (A) is one or two or more selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.
(3) The cosmetic preparation according to (1), wherein the component (A) is one component selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.
(4) The cosmetic preparation according to (1), wherein the component (A) is two components selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane, and the two components are any of combinations of the components (A-1) and (A-2), the components (A-1) and (A-3), and the components (A-2) and (A-3).
(5) The cosmetic preparation according to (1), wherein the component (A) includes three components (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.
(6) The cosmetic preparation according to (1), wherein the component (A) is two components selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane, the two components are the components (A-1) and (A-2) or the components (A-2) and (A-3), and a content of the component (A-2) is 50 mass% or more.
(7) The cosmetic preparation according to (1), wherein the component (A) is two components selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane, the two components are the components (A-1) and (A-2) or the components (A-2) and (A-3), and a content of the component (A-2) is 60 mass% or more.
(8) The cosmetic preparation according to (1), wherein the component (A) is two components selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane, the two components are the components (A-1) and (A-2) or the components (A-2) and (A-3), and a content of the component (A-2) is 70 mass% or more.
(9) The cosmetic preparation according to (1), wherein the component (A) is used as a mixture of the components (A-1) and (A-3), and an amount of the component (A-1) is 20 to 80 mass% and an amount of the component (A-3) is 80 to 20 mass% in a total amount of 100 mass% of the components (A-1) and (A-3).
(10) The cosmetic preparation according to (1), wherein the component (A) is used as a mixture of the components (A-1) and (A-3), and an amount of the component (A-1) is 30 to 70 mass% and an amount of the component (A-3) is 70 to 30 mass% in a total amount of 100 mass% of the components (A-1) and (A-3).
(11) The cosmetic preparation according to (1), wherein the component (A) is used as a mixture of the components (A-1) and (A-3), and an amount of the component (A-1) is 40 to 60 mass% and an amount of the component (A-3) is 60 to 40 mass% in a total amount of 100 mass% of the components (A-1) and (A-3).
(12) The cosmetic preparation according to (1), wherein the component (A) is used as a mixture of the components (A-1), (A-2), and (A-3), and an amount of the component (A-1) is 5 to 80 mass%, an amount of the component (A-2) is 10 to 70 mass%, and an amount of the component (A-3) is 10 to 70 mass% in a total of 100 mass% of the components (A-1), (A-2), and (A-3).
(13) The cosmetic preparation according to (1), wherein the component (A) is used as a mixture of the components (A-1), (A-2), and (A-3), and an amount of the component (A-1) is 10 to 70 mass%, an amount of the component (A-2) is 20 to 60 mass%, and an amount of the component (A-3) is 20 to 60 mass% in a total of 100 mass% of the components (A-1), (A-2), and (A-3).
(14) The cosmetic preparation according to (1), wherein the component (A) is used as a mixture of the components (A-1), (A-2), and (A-3), and an amount of the component (A-1) is 20 to 60 mass%, an amount of the component (A-2) is 20 to 50 mass%, and an amount of the component (A-3) is 20 to 50 mass% in a total of 100 mass% of the components (A-1), (A-2), and (A-3).
(15) The cosmetic preparation according to any one of (1) to (14), wherein the silicone resin of the component (B) is used as a mixture of a silicone resin composed mainly of R₃SiO_{1/2} unit and RSiO_{3/2} unit and a silicone resin composed mainly of R₃SiO_{1/2} unit and SiO_{4/2} unit.
(16) The cosmetic preparation according to any one of (1) to (14), wherein the silicone resin of the component (B) is used as a mixture of a silicone resin composed mainly of R₃SiO_{1/2} unit and RSiO_{3/2} unit and a silicone resin composed mainly of R₃SiO_{1/2} unit and SiO_{4/2} unit, and
   R in the above constituent units is a substituted or unsubstituted monovalent hydrocarbon group selected from alkyl groups such as a methyl group, an ethyl group, a propyl group, and a butyl group, alkenyl groups such as a vinyl group and an allyl group, aryl groups such as a phenyl group and a tolyl group, cycloalkyl groups such as a cyclohexyl group and a cyclooctyl group, or groups formed by the substitution of a halogen atom, a cyano group, an amino group, or the like for a hydrogen atom bonded to a carbon atom of these groups, a cyanomethyl group, a γ-aminopropyl group, and an N-(β-aminoethyl)-γ-aminopropyl group.
(17) The cosmetic preparation according to any one of (1) to (14), wherein the silicone resin of the component (B) is used as a mixture of a silicone resin composed mainly of R₃SiO_{1/2} unit and RSiO_{3/2} unit and a silicone resin composed mainly of R₃SiO_{1/2} unit and SiO_{4/2} unit, and
   R in the above constituent units is a substituted or unsubstituted monovalent hydrocarbon group selected from a methyl group, an ethyl group, or a phenyl group.
(18) The cosmetic preparation according to any one of (1) to (14), wherein the silicone resin of the component (B) is a silicone resin composed of a combination of R₃SiO_{1/2} unit, R₂SiO_{2/2} unit, RSiO_{3/2} unit, and SiO_{4/2} unit.
(19) The cosmetic preparation according to any one of (1) to (14), wherein the silicone resin of the component (B) is a silicone resin composed of a combination of R₃SiO_{1/2} unit, R₂SiO_{2/2} unit, RSiO_{3/2} unit, and SiO_{4/2} unit, and a content of R₂SiO_{2/2} unit as a constituent is 30 mol% or less.
(20) The cosmetic preparation according to any one of (1) to (14), wherein the silicone resin of the component (B) has a thermal softening point of 60°C or more.
(21) The cosmetic preparation according to any one of (1) to (14), wherein the silicone resin of the component (B) has a thermal softening point of 70°C or more.
(22) The cosmetic preparation according to any one of (1) to (21), wherein a total content of the components (A) and (B) in the cosmetic preparation is 1 to 50 mass%.
(23) The cosmetic preparation according to any one of (1) to (21), wherein a total content of the components (A) and (B) in the cosmetic preparation is 3 to 30 mass%.
(24) The cosmetic preparation according to any one of (1) to (21), wherein a total content of the components (A) and (B) in the cosmetic preparation is 3 to 20 mass%.
(25) The cosmetic preparation according to any one of (1) to (24), wherein a content of each component in a total content of 100 mass% of the components (A) and (B) in the cosmetic preparation varies depending on a formulation of the cosmetic preparation.
(26) The cosmetic preparation according to any one of (1) to (24), wherein a content of the component (A) is 50 to 95 mass% and a content of the component (B) is 5 to 50 mass% in a total content of 100 mass% of the components (A) and (B) in the cosmetic preparation.
(27) The cosmetic preparation according to any one of (1) to (24), wherein a content of the component (A) is 60 to 90 mass% and a content of the component (B) is 10 to 40 mass% in a total content of 100 mass% of the components (A) and (B) in the cosmetic preparation.
(28) The cosmetic preparation according to any one of (1) to (27), wherein in addition to the components (A) and (B), the preparation further contains an oily component selected from solid paraffin, ceresin, beeswax, carnauba wax, wood wax, candelilla wax, stearic acid, cetanol, cholesterol, microcrystalline wax, petroleum jelly, lanolin, partially hydrogenated oils, squalene, liquid paraffin, castor oils, diglycerides, triglycerides, higher fatty acids, higher alcohols, perfluoropolyethers, perfluorodecalin, perfluorooctane, ester oils, octyldodecyl myristate, octyldodecyl oleate, neopentyl glycol dioctanoate, or combinations of these.
(29) The cosmetic preparation according to any one of (1) to (28), wherein in addition to the components (A) and (B), the preparation further contains an aqueous component selected from glycerin, sorbitol, propylene glycol, 1,3-butylene glycol, polyethylene glycol, carboxyvinyl polymer, cellulose derivatives, sodium alginate, ethanol, or combinations of these.
(30) The cosmetic preparation according to any one of (1) to (29), wherein in addition to the components (A) and (B), the preparation further contains, as a surfactant, one or two or more selected from the following,
   the following anionic emulsifiers: sodium alkyl benzene sulfonates such as sodium hexyl benzene sulfonate, sodium octyl benzene sulfonate, sodium decyl benzene sulfonate, sodium dodecyl benzene sulfonate, sodium cetyl benzene sulfonate, and sodium myristyl benzene sulfonate; sodium alkyl naphthyl sulfonates such as sodium butyl naphthyl sulfonate; polyoxyethylene monoalkyl ether sulfate sodium salts such as sodium polyoxyethylene octyl ether sulfate, sodium polyoxyethylene decyl ether sulfate, and sodium polyoxyethylene icosyl ether sulfate; polyoxyethylene mono(alkylphenyl)ether sulfate sodium salts such as sodium polyoxyethylene decylphenyl ether sulfate, and others,
   the following cationic emulsifiers: quaternary ammonium salts such as octyl trimethyl ammonium chloride, dodecyl trimethyl ammonium chloride, hexadecyl trimethyl ammonium chloride, beef tallow trimethyl ammonium chloride, coconut oil trimethyl ammonium chloride, octyl dimethyl benzyl ammonium chloride, decyl dimethyl benzyl ammonium chloride, and dioctadecyl dimethyl ammonium chloride, and others, and
   the following nonionic emulsifiers: glycerin fatty acid esters such as glyceryl monolaurate, glyceryl monomyristate, glyceryl monostearate, and glyceryl monooleate; polyglycerin fatty acid esters, sorbitan fatty acid esters, polyoxyethylene glycerin fatty acid esters, polyoxyethylene sorbitan fatty acid esters, and polyoxyethylene fatty acid esters having a lauric acid residue, a myristic acid residue, a stearic acid residue, or an oleic acid residue; polyoxyethylene alkyl ethers such as polyoxyethylene lauryl ether, polyoxyethylene cetyl ether, polyoxyethylene stearyl ether, and polyoxyethylene oleyl ether, and others.
(31) The cosmetic preparation according to any one of (1) to (30), wherein in addition to the components (A) and (B), the preparation further contains, as a powder, one or more selected from the following:
   inorganic powders including talc, kaolin, sericite, white mica, synthetic mica, golden mica, red mica, black mica, lithia mica, vermiculite, magnesium carbonate, calcium carbonate, diatomaceous earth, magnesium silicate, calcium silicate, aluminum silicate, barium silicate, barium sulfate, strontium silicate, metal salts of tungstic acid, silica, hydroxyapatite, zeolite, boron nitride, and ceramic powder;
   organic powders including nylon powder, polyethylene powder, benzoguanamine powder, ethylene tetrafluoride powder, distyrene benzene pinhole polymer powder, and microcrystalline cellulose;
   powders of silicone rubber and silicone resin; inorganic white pigments such as titanium oxide and zinc oxide; inorganic red pigments such as an iron oxide (iron oxide red) and iron titanate; inorganic brown pigments such as γ-iron oxide and others; inorganic yellow pigments including yellow iron oxide and yellow ocher;
   inorganic black pigments including black iron oxide and carbon black;
   inorganic purple pigments including mango violet and cobalt violet;
   inorganic green pigments including chromium oxide, chromium hydroxide, and cobalt titanate;
   inorganic blue pigments including ultramarine and Prussian blue;
   pearl pigments including titanium oxide coated mica, titanium oxide coated bismuth oxychloride, bismuth oxychloride, titanium oxide coated talc, fish scale flakes, and colored titanium oxide coated mica;
   metal powder pigments including aluminum powder and copper powder;
   organic pigments such as Red No.201, Red No.202, Red No.204, Red No.205, Red No.220, Red No.226, Red No.228, Red No.405, Orange No.203, Orange No.204, Yellow No.205, Yellow No.401, and Blue No.404, and others; organic pigments including zirconium, barium, or aluminum lakes of Red No.3, Red No.104, Red No.106, Red No.227, Red No.203, Red No.401, Red No.505, Orange No.205, Yellow No.4, Yellow No.5, Yellow No.202, Yellow No.203, Green No.3, and Blue No.1; and
   natural pigments including chlorophyll and β-carotene.
(32) The cosmetic preparation according to any one of (1) to (31), wherein in addition to the components (A) and (B), the preparation further contains one or more selected from coloring agents including organic pigments and organic dyes, preservatives, antioxidants, colorants, thickeners, pH adjusters, fragrances, UV absorbers, moisturizers, blood circulation promoters, cooling agents, antiperspirants, bactericides, skin activators, and water.
(33) The cosmetic preparation according to any one of (1) to (32), wherein in addition to the components (A) and (B), the preparation further contains one or more selected from oil components including various types of hydrocarbons, higher fatty acids, oils and fats, esters, higher alcohols, waxes, and silicone oils, including squalane, liquid paraffin, isoparaffin, petroleum jelly, microcrystalline wax, ozokerite, ceresin, myristic acid, palmitic acid, stearic acid, oleic acid, isostearic acid, cetyl alcohol, stearyl alcohol, hexadecyl alcohol, oleyl alcohol, cetyl-2-ethylhexanoate, 2-ethylhexyl palmitate, 2-octyldodecyl myristate, 2-octyldodecyl gum ester, neopentyl glycol-2-ethylhexanate, isooctyl acid triglyceride, 2-octyldodecyl oleate, isopropyl myristate, isopropyl palmitate, isostearic acid triglyceride, coconut oil fatty acid triglycerides, olive oil, avocado oil, beeswax, myristyl myristate, mink oil, and lanolin.
(34) The cosmetic preparation according to any one of (1) to (33), wherein the preparation is in any of liquid formulations, powder formulations, solid formulations, and emulsion formulations.
(35) The cosmetic preparation according to any one of (1) to (33), wherein the preparation is a skin cosmetic preparation or a makeup cosmetic preparation selected from cosmetic primers, foundations, sunscreens, lipsticks, eyeliners, mascaras, and skin creams.

### Examples

The present invention is illustrated with examples below, but the present invention is not limited thereto.

The components used in the examples and comparative examples are as follows. Note that, in the following, "synthetic product" means that the product was synthesized by the present inventors.

### Component (A)

1,1,1,3,5,5,5-heptamethyl-3-dodecyltrisiloxane: synthetic product
1,1,1,3,5,5,5-heptamethyl-3-decyltrisiloxane: synthetic product
1,1,1,3,5,5,5-heptamethyl-3-tetradecyltrisiloxane: synthetic product

### Component (B)

Trimethylsiloxysilicate: SR 1000 manufactured by Momentive Performance Materials Inc., thermal softening point 100°C or more, a = 1.5 in the aforementioned average unit formula

Polymethylsilsesquioxane: SilForm Flexible Resin manufactured by Momentive Performance Materials Inc., thermal softening point 90°C, a = 1.75 in the aforementioned average unit formula

### (Oily component)

1,1,1,3,5,5,5-heptamethyl-3-octyltrisiloxane: Silsoft 034 manufactured by Momentive Performance Materials Inc.

Polydimethylsiloxane with a viscosity of 5 cSt: Element14 PDMS 5-JC manufactured by Momentive Performance Materials Inc.

Glyceryl tri(caprylate/caprate), PEG/PPG-20/15 dimethicone: HARMONIE Mesh emulsifier manufactured by Momentive Performance Materials Inc.

Diphenyl dimethicone: SilShine 150 manufactured by Momentive Performance Materials Inc.

### (Powder component)

Silica: HARMONIE Luxe 4 powder manufactured by Momentive Performance Materials Inc.

Boron nitride: CCS 102-JA manufactured by Momentive Performance Materials Inc.

Hydrophobically surface-treated titanium oxide: SI01-2 CR-50 manufactured by DAITO KASEI KOGYO CO., LTD.

Hydrophobically surface-treated iron oxide (yellow): SI-2 YELLOW LL-100P manufactured by DAITO KASEI KOGYO CO., LTD.

Hydrophobically surface-treated iron oxide (red): SI-2 RED R-516 manufactured by DAITO KASEI KOGYO CO., LTD.

Hydrophobically surface-treated iron oxide (black): SI-2 BLACK BL-100P manufactured by DAITO KASEI KOGYO CO., LTD.

### (Aqueous component)

Butylene glycol: reagent
Glycerin: reagent
Sodium chloride: reagent

### Examples 1 to 6 and comparative examples 1 and 2 (BB cream)

Phase I components, phase II components, and phase III components shown in Table 1 were each stirred until the components became uniform to prepare a mixture of each phase. Next, the mixture of the phase II was added to the mixture of the phase I and stirred until they became uniform, and subsequently the mixture of the phase III was added thereto and stirred until they became uniform to obtain BB creams of examples and comparative examples.

The obtained cosmetic primer creams were evaluated for spreading on the skin, non-stickiness, softness to the touch, and makeup longevity by the methods below. The results are shown in Table 1. Note that, in Table 1, the numerical values of components are each expressed as mass%, and water is in adjusting amounts which make a total of 100 mass%.

### (Spreading on the skin)

The spreading on the skin when an appropriate amount (about 1 g) of each of the cosmetic preparations of the examples and comparative examples was placed on the hand, and then applied to the face and spread with fingers was evaluated. In other words, ten panelists made evaluations out of 5 with comparative example 1 as 3 (5: superior, 4: slightly superior, 3: equal, 2: slightly inferior, 1: inferior), and the average score thereof was as shown in Table 1.

### (Sticky feeling)

After the application to the skin, whether the skin felt non-sticky and refreshing was evaluated. In other words, ten panelists made evaluations out of 5 with comparative example 1 as 3 (5: superior, 4: slightly superior, 3: equal, 2: slightly inferior, 1: inferior), and the average score thereof was as shown in Table 1.

### (Softness to the touch)

After the application to the skin and drying, softness to the touch with no tight feeling of the skin was evaluated. In other words, ten panelists made evaluations out of 5 with comparative example 1 as 3 (5: superior, 4: slightly superior, 3: equal, 2: slightly inferior, 1: inferior), and the average score thereof was as shown in Table 1.

### (Makeup longevity)

The makeup longevity six hours after the application to the skin was evaluated. In other words, ten panelists made evaluations out of 5 with comparative example 1 as 3 (5: superior, 4: slightly superior, 3: equal, 2: slightly inferior, 1: inferior), and the average score thereof was as shown in Table 1.

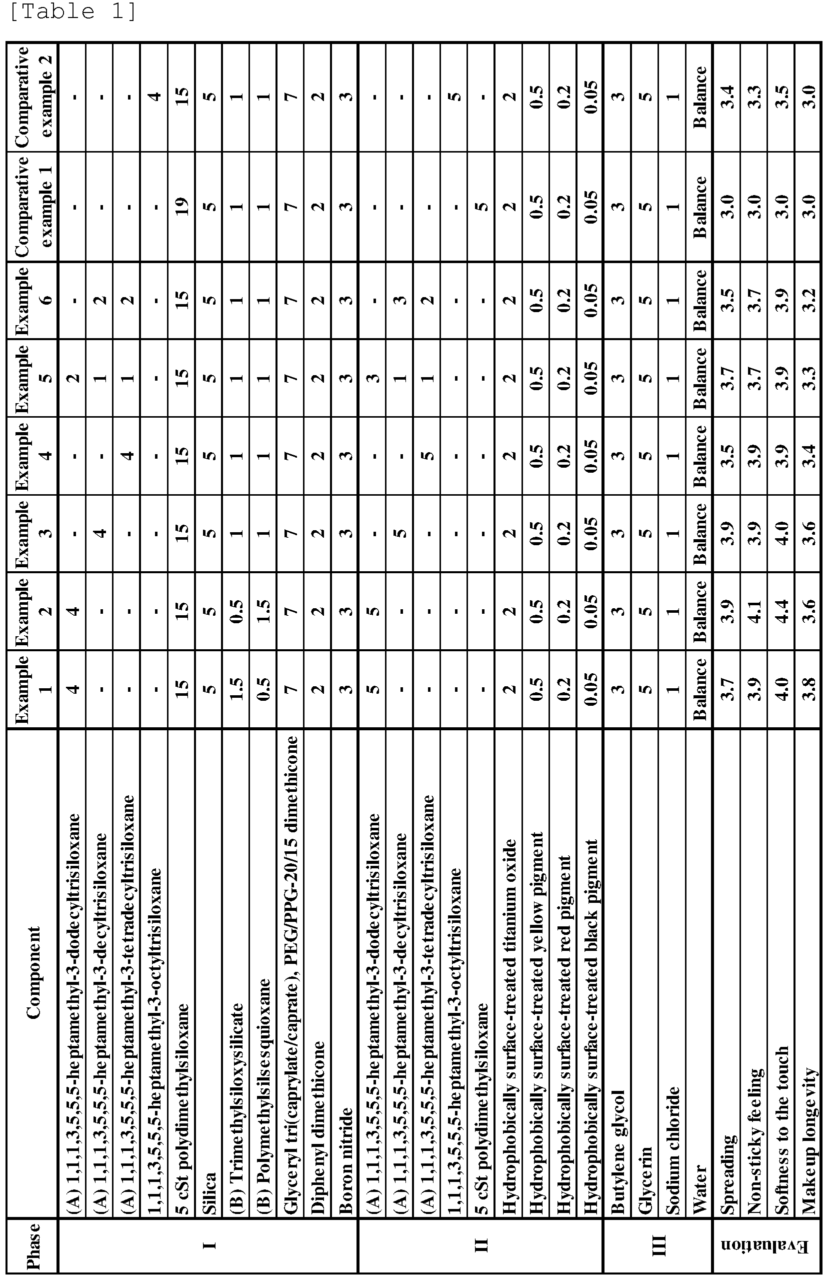

The cosmetic preparation of the present invention attained excellent evaluations in all the evaluations shown in Table 1 due to the combined use of the components (A) and (B).

The cosmetic preparation of the present invention can provide a silky feel with no sticky feeling, good water resistance due to excellent water repellency, and others when used as a skin cosmetic preparation, and can provide even, good spreading, excellent makeup longevity due to excellent water repellency, water resistance, or the like, a good appearance due to an excellent glow and coloration of pigments, and a smooth rubbing-in feeling, and others when used as a makeup cosmetic preparation.

### Example 7 (liquid foundation)

| | | |
|---|---|---|
| **(1)** | **(A) 1,1,1,3,5,5,5-heptamethyl-3-dodecyltrisiloxane** | **4.00** |
| **(2)** | **Diphenyl dimethicone** | **5.00** |
| **(3)** | **(B) Polymethylsilsesquioxane** | **1.00** |
| **(4)** | **(B) Trimethylsiloxysilicate** | **1.00** |
| **(5)** | **Decamethylcyclopentasiloxane** | **2.00** |
| **(6)** | **Dimethicone, cetearyl dimethicone crosspolymer** | **4.00** |
| **(7)** | **Glyceryl tri(caprylate/caprate), PEG/PPG-20/15 dimethicone** | **5.00** |
| **(8)** | **(A) 1,1,1,3,5,5,5-heptamethyl-3-dodecyltrisiloxane** | **7.00** |
| **(9)** | **Silica** | **5.00** |
| **(10)** | **Hydrophobically surface-treated titanium oxide** | **4.50** |
| **(11)** | **Hydrophobically surface-treated iron oxide (yellow)** | **0.28** |
| **(12)** | **Hydrophobically surface-treated iron oxide (red)** | **0.18** |
| **(13)** | **Hydrophobically surface-treated iron oxide (black)** | **0.05** |
| **(14)** | **Water** | **54.99** |
| **(15)** | **Glycerin** | **5.00** |
| **(16)** | **Sodium chloride** | **1.00** |
| **Total** | | **100.00 mass %** |

### (Preparation method)

After the above components (1) to (7) and components (8) to (13) were each mixed until they became uniform, the uniform mixture of the components (1) to (7) and the uniform mixture of the components (8) to (13) were mixed at 70°C for 30 minutes to obtain a uniform mixture of the components (1) to (13).

Subsequently, the components (14) to (16) uniformly dissolved at 70°C were added and mixed to the uniform mixture of the components (1) to (13), and then stirred with a homogenizer at 2,000 r/m for 5 minutes to obtain a liquid foundation.

### (Evaluation)

The obtained liquid foundation was a uniform dispersion without lumps or the like, was smooth and spread well when applied to the skin, and was soft and smooth to the touch after drying.

### Example 8 (skin cream)

| | | |
|---|---|---|
| **(1)** | **Water** | **64.37** |
| **(2)** | **Glycerin (reagent)** | **3.00** |
| **(3)** | **Dissolvine NA2-S manufactured by Lion Corporation: EDTA 2Na** | **0.05** |
| **(4)** | **EMALEX SEG-07 manufactured by NIHON EMULSION Co., Ltd.: self-emulsifying glyceryl stearate** | **2.50** |
| **(5)** | **KALCOL 6850 manufactured by Kao Corporation: cetostearyl alcohol** | **2.50** |
| **(6)** | **SF1642*: both ends C30-45 alkyl-modified silicone wax** | **1.20** |
| **(7)** | **(A) 1,1,1,3,5,5,5-heptamethyl-3-dodecyltrisiloxane (synthetic)** | **8.00** |
| **(8)** | **(B) SR 1000*: polymethylsiloxysilicate** | **0.50** |
| **(9)** | **(B) SilForm Flexible Resin*: polymethylsilsesquioxane** | **0.50** |
| **(10)** | **ECO OIL RS manufactured by KOKYU ALCOHOL KOGYO CO., LTD.: jojoba oil** | **1.00** |
| **(11)** | **Star Shea Butter Refined manufactured by Nikko Chemicals Co., Ltd.: shea butter** | **2.00** |
| **(12)** | **Vitamin E Acetate Care manufactured by BASF SE: tocopherol acetate** | **0.10** |
| **(13)** | **AQUPEC SW-750E manufactured by SUMITOMO SEIKA CHEMICALS CO., LTD.: carboxyvinyl polymer** | **0.20** |
| **(14)** | **Glycerin (reagent)** | **2.00** |
| **(15)** | **Water** | **8.00** |
| **(16)** | **Aminomethyl propanol (reagent)** | **0.20** |
| **(17)** | **Velvesil E Gel PMF*: silicone gel emulsion** | **2.00** |
| **(18)** | **Sodium hyaluronate (reagent)** | **0.20** |
| **(19)** | **Phenoxy ethanol (reagent)** | **0.50** |
| **(20)** | **Fragrance** | **0.20** |

**All the numerical values are expressed as mass %, and total up to 100 mass %.**

**Those followed by an asterisk (*) are manufactured by Momentive Performance Materials Inc.**

### (Preparation method)

The above components (1) to (3) were heated to 75°C while stirring them well. The components (4) to (12) were heated to 70°C while stirring them well. The mixture of the components (4) to (12) was added to the mixture of the components (1) to (3), and they were stirred with a homogenizer (950 rpm) for 2 minutes and cooled to 40°C. After a mixture of the components (13) to (15) stirred until they became uniform was added to the mixed liquid of the components (1) to (12) and stirred, the component (16) was added and stirred. The components (17) to (20) stirred until they became uniform were added thereto and stirred to obtain a skin cream.

### (Evaluation)

The obtained skin cream was uniform and spread well, had a smooth feel with no tight feeling or stiff feeling even after drying, and made a moist, moisturized feeling felt even half a day after the application.

### Industrial Applicability

The cosmetic preparation of the present invention can be in any of liquid formulations, powder formulations, solid formulations, and emulsion formulations, and can be utilized as a skin cosmetic preparation or a makeup cosmetic preparation selected from cosmetic primers, foundations, sunscreens, lipsticks, eyeliners, mascaras, skin creams, and others.

## Claims

1. A cosmetic preparation comprising the following components (A) and (B):
(A) one or two or more selected from 1,1,1,3,5,5,5-heptamethyl-3-alkyl (C10-14) trisiloxanes; and
(B) a silicone resin having an average unit formula of RₐSiO_{(4-a)/2} and a thermal softening point of 50°C or more, wherein R represents the same or different substituted or unsubstituted monovalent hydrocarbon groups, and a is a number from 1.2 to 2.

2. The cosmetic preparation according to claim 1, wherein the component (A) is any one component selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.

3. The cosmetic preparation according to claim 1, wherein the component (A) is any one of combinations of components (A-1) and (A-2), components (A-1) and (A-3), and components (A-2) and (A-3) selected from (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.

4. The cosmetic preparation according to claim 1, wherein the component (A) comprises three components (A-1) 1,1,1,3,5,5,5-heptamethyl-3-decyltrisiloxane, (A-2) 1,1,1,3,5,5,5-heptamethyl-3-dodecyltrisiloxane, and (A-3) 1,1,1,3,5,5,5-heptamethyl-3-tetradecyltrisiloxane.

5. The cosmetic preparation according to claim 1, wherein the silicone resin (B) is a silicone resin composed mainly of R₃SiO_{1/2} unit and RSiO_{3/2} unit and a silicone resin composed mainly of R₃SiO_{1/2} unit and SiO_{4/2} unit.

6. The cosmetic preparation according to claim 1, wherein the preparation comprises the components (A) and (B) in an amount of 1 to 50 mass% in total.

7. The cosmetic preparation according to claim 1, wherein a content of the component (A) is 50 to 95 mass% and a content of the component (B) is 5 to 50 mass% in a total content of 100 mass% of the components (A) and (B) in the cosmetic preparation.

8. The cosmetic preparation according to any one of claims 1 to 7, wherein the preparation is a skin cosmetic preparation or a makeup cosmetic preparation selected from cosmetic primers, foundations, sunscreens, lipsticks, eyeliners, mascaras, and skin creams.
